# EUROPÄISCHE PATENTSCHRIFT

(11) **EP 2 560 554 B1**
(45) Veröffentlichungstag und Bekanntmachung des Hinweises auf die Patenterteilung: **05.07.2017**
(21) Anmeldenummer: 11721250.6
(22) Anmeldetag: 19.04.2011
(51) Int. Cl.: A61B 17/00, A61B 17/02

(54) **INVASIVES INSTRUMENT ZUR BEARBEITUNG VON GEFÄSSEN**
INVASIVE INSTRUMENT FOR TREATING VESSELS
INSTRUMENT INVASIF POUR LE TRAITEMENT DE VAISSEAUX

(30) Priorität: 23.04.2010 DE 102010028167
(43) Veröffentlichungstag der Anmeldung: 27.02.2013
(73) Patentinhaber: W.O.M. World of Medicine GmbH, 10587 Berlin (DE)
(72) Erfinder: FELS, Esther, 13347 Berlin (DE); WILKE, Marsha, 10781 Berlin (DE); GELBERT, Nils, 12555 Berlin (DE); TSCHEPE, Johannes, 10781 Berlin (DE); SCHÖNBORN, Karl-Heinz, 12355 Berlin (DE)
(74) Vertreter: Maikowski & Ninnemann Patentanwälte Partnerschaft mbB
(86) Internationale Anmeldenummer: PCT/EP2011/056222
(87) Internationale Veröffentlichungsnummer: WO 2011/131660

(56) Entgegenhaltungen:
- EP-A1- 2 116 189
- EP-A2- 1 323 373
- EP-A2- 1 323 373
- WO-A1-00/40139
- WO-A1-01/62183
- WO-A1-2005/094741
- WO-A1-2006/046950
- WO-A1-2011/082287
- WO-A2-02/19945
- WO-A2-03/013367
- US-A- 5 722 934
- US-A- 5 984 937
- US-A1- 2002 099 259
- US-A1- 2005 010 242
- US-A1- 2011 046 624
- US-B1- 6 264 670

## Beschreibung

Die Erfindung betrifft ein invasives Instrument zur Bearbeitung von Gefäßen nach Anspruch 1.
Es ist bekannt, die für einen Bypass verwendeten körpereigenen Gefäße, also Venen und Arterien, in einem invasiven, häufig auch minimal invasiven Verfahren unter endoskopischer Kontrolle zu entnehmen. Dieses Verfahren nennt sich Endoscopic Vessel Harvesting (EVH). Bei diesem Verfahren kommen speziell entwickelte Instrumente zur Anwendung.
Bevor es zur eigentlichen Gefäßentnahme kommt, muss das Gefäß von dem das Gefäß umschließenden Gewebe getrennt werden.
Zunächst wird mittels eines kleinen Schnitts in unmittelbarer Nähe des zu entnehmenden Gefäßes, beispielsweise zur Entnahme der Vena Saphena Magna im Bereich des Kniegelenkspaltes, ein Zugang geschaffen. Anschließend erfolgen die Einführung eines geeignet geformten Instrumentes und die schrittweise Trennung (Dissektion) mittels Vorschieben entlang der Gefäßachse unter visueller Kontrolle und / oder endoskopischer Sicht.
Die bekannten Instrumente können z. B. mit einem Endoskop und / oder einer Kamera verwendet werden. Aus der Patentschrift US 6,042,538 ist bekannt, dass sich eine Endoskop-Optik in das Instrument integrieren lässt. Hierbei kann das Instrument sowohl als Einweg- als auch Mehrwegprodukt ausgebildet werden. Bei einem Mehrwegprodukt kann die Endoskop-Optik auch unlösbar in das Instrument integriert werden.
Dem Stand der Technik entsprechend wird das Endoskop, welches häufig in dem Instrument integriert ist, mit einer Videokamera verbunden, so dass der Behandelnde sein Vorgehen auf einem Bildschirm beobachten kann.
Aus der Patentschrift US 5,928,138 ist bekannt, dass eine Spitze aus visuell transparentem Material gefertigt werden kann, damit dem Operateur während der Dissektion über das Endoskop eine gute Sicht nach vorne gewährleistet ist. Solch eine transparente Spitze wird auch als optischer Dissektor bezeichnet.

Das zu entnehmende Gefäß ist mit weiteren, kleineren peripheren Gefäßen verbunden und muss nach der Dissektion vom umliegenden Gewebe auch von diesen getrennt werden.

Dies erfolgt nach dem Stand der Technik durch in dem Instrument integrierte Schneidvorrichtungen. Aus der Patentschrift US 6,022,313 ist bekannt, dass solch eine Schneidvorrichtung, z. B. mit einer Schere, die in einem Arbeitskanal des Instrumentes vorgeschoben wird, ausgeführt werden kann.

Aus den Patentschriften US 5,928,138 und US 6,022,313 ist bekannt, dass sich im Instrument Vorrichtungen zur Führung des zu entnehmenden Gefäßes integrieren lassen.

Aus der Patentschrift US 7,645,289 ist bekannt, dass eine transparente Spitze sich entlang der Längsachse des Instrumentes mittels eines am Handgriff integrierten Manipulators verschieben lässt. Darüber hinaus ist aus der Patentschrift US 7,645,289 bekannt, dass die Spitze dem Instrument zugewandte Seite der Spitze mit einer Aussparung versehen ist. Die Formgestaltung der Aussparung erlaubt die Aufnahme und / oder Fixierung der abzutrennenden peripheren Gefäße. Auch aus der EP 1 323 373 A2 ist eine axial bewegliche Spitze bekannt, die so ausgestaltet ist, dass sie ein Blutgefäß in einer Position halten kann.

Es ist ferner bekannt, die endoskopisch-gestützte Gefäßentnahme mittels CO₂ Gas Insufflation zu unterstützen. Es ist bekannt, dass CO₂-Gas durch Hohlräume und / oder Kanäle eines Instrumentes für endoskopische Eingriffe gezielt in den Körper geleitet werden kann.

Es besteht die Aufgabenstellung, ein Instrument zur invasiven, vorzugsweise endoskopischen Bearbeitung, insbesondere zur Entnahme von Gefäßen zu schaffen, mit dem insbesondere umliegendes Gewebe in-situ effizient bearbeitet werden kann.

Diese Aufgabe wird durch ein invasives Instrument zur Bearbeitung eines Gefäßes, das ggf. Nebengefäße aufweist, mit einer zur Dissektion von Gewebe ausgebildeten Spitze am distalen Ende eines in den Körper einzuführenden Schaftes gelöst. Dabei weist die Spitze mindestens eine Ausnehmung, insbesondere eine Gefäßaufnahme auf, die so angeordnet und ausgebildet ist, dass im Betriebszustand des Instruments stets mindestens eine Funktionseinheit des Schaftes freigelassen ist, so dass Arbeiten am Gefäß, einem Nebengefäß und/oder am umliegenden Gewebe mit oder durch die Funktionseinheit möglich sind. Durch das Freihalten einer Funktionseinheit (z. B. einem Arbeitskanal für eine Schneidvorrichtung) ist es für den Behandelnden einfacher, in-situ zu arbeiten, ohne z. B. die Spitze wechseln zu müssen. Mit einem dermaßen ausgeführten Instrument können mehrere Funktionen gleichzeitig durchgeführt werden, wie z.B. das Halten des Gefäßes (d.h. des Gefäßaufnahme für das Hauptgefäß) und die gleichzeitige Bearbeitung am Nebengefäß und / oder am umliegenden Gewebe.

Mit Vorteil weist die Spitze mindestens teilweise einen kegelförmigen Bereich, einen prismatischen Bereich, einen Bereich in der Form einer Delfinnase, einen Bereich mit der Form eines Kegelstumpfs und/oder einen Bereich mit der Form eines Dreikants auf. Mit diesen Formgebungen lässt sich eine effiziente Dissektion des umliegenden Gewebes erreichen.

Ferner ist das zu bearbeitende Gefäß mit dem mindestens einen Gefäßaufnahmemittel in einer räumlichen Position fixierbar, wobei die Spitze mittels eines Führungselementes relativ zum Schaft bewegbar ist. Der Behandelnde kann nach der Einführung des invasiven Gerätes in den Körper damit z. B. in-situ eine Präparation des Gefäßes vornehmen.

Dabei ist die Spitze in axialer Richtung verschiebbar, so dass im Eingriff ein im mindestens einen Gefäßaufnahmemittel angeordnetes Gefäß von der Funktionseinheit gezielt beabstandbar ist.

Dazu ist es auch vorteilhaft, wenn die Spitze um bis zu 180° um die Längsachse verschwenkbar ist, so dass im Eingriff ein im mindestens einen Gefäßaufnahmemittel angeordnetes Gefäß von insbesondere einer Schneidvorrichtung gezielt beabstandbar ist. Durch die Herstellung einer möglichst großen Distanz zwischen Gefäß und z. B. einer Schneidvorrichtung wird die Gefahr einer Schädigung des Gefäßes minimiert. Auch für andere Funktionseinheiten, z. B. ein Endoskop, kann die Beabstandung sinnvoll sein, da damit in-situ ein großer Blickbereich einstellbar ist.

Zudem ragt die Endoskopspitze aus der Instrumentenspitze heraus und in die eingefahrene transparente Spitze hinein.

Für die Handhabung der Spitze ist es vorteilhaft, wenn ein Manipulator, insbesondere an einem Handgriff angeordnet ist. Mit diesem kann eine gezielte Verschiebung in axialer Richtung und/oder eine Rotation der Spitze erfolgen.

Ferner ist es vorteilhaft, wenn das Führungselement im Wesentlichen stabförmig ausgebildet ist und an einer Stelle der Spitze befestigt ist, die dem Gefäßaufnahmemittel gegenüberliegt. Durch eine möglichst große Beabstandung zwischen dem Drehpunkt der Spitze und dem Gefäßaufnahmemittel ist es möglich, das Gefäßaufnahmemittel weit von Funktionseinheiten im Schaft wegzudrehen. Es ist auch vorteilhaft, dass der Führungskanal und der Arbeitskanal auf gegenüberliegenden Seiten des Schaftes angeordnet sind.

Funktionseinheiten, die im Schaft und/oder im Handgriff des Instruments angeordnet sein können, sind mindestens als ein Endoskopkanal, mindestens als ein Arbeitskanal zur Aufnahme einer Schneidvorrichtung, mindestens als ein Führungskanal zur Aufnahme eines Führungselementes, mindestens als ein Spülkanal und/oder mindestens als ein Insufflationskanal ausgebildet.

Auch ist es vorteilhaft, wenn die Spitze mit der Ausnehmung derart gestaltet ist, dass sich die Schneidvorrichtung und/oder ein Endoskop in axial eingefahrener Position und/oder auch axial ausgefahrener Position an der Spitze vorbeischieben lässt.

Vorteilhafterweise ist die Distanz zwischen ausgefahrener Spitze und Instrumentenspitze stufenlos oder in diskreten Schritten einstellbar. Damit kann eine besonders effiziente Präparation des Gefäßes erreicht werden.

Es ist vorteilhaft, wenn der Handgriff und/oder der Schaft als Mehrweginstrument ausgeführt sind. Auch ist es vorteilhaft, wenn die Spitze, die Führungsstange und/oder der Manipulator (13) als Einwegkomponenten ausgeführt sind.

Für die in-situ Arbeit ist es vorteilhaft, wenn im Schaft mindestens ein Kanal zur Leitung von Spülflüssigkeit und/oder zur Absaugung unerwünschter Flüssigkeiten integriert ist.

Insbesondere für Reinigungszwecke ist es vorteilhaft, wenn sich Gas, insbesondere CO₂-Gas für die Insufflation in einem Insufflationskanal bis zur Instrumentenspitze leiten lässt.

Besonders vorteilhaft ist es, wenn der Insufflationskanal und/oder der Spülkanal relativ zur Endoskopspitze derart angeordnet ist, dass sich mit dem Gas und/oder der Spülflüssigkeit die Endoskopspitze reinigen lässt, insbesondere indem der Gasstrom und/oder Flüssigkeitsstrom an der Rückseite der Spitze umgelenkt und auf die Endoskopspitze gerichtet wird.

In einer weiteren Fortbildung ist an der Instrumentenspitze eine Videokamera angeordnet, wobei die Videokamera einen Videosensor, z. B. einen CCD- oder CMOS-Sensor, nebst dahinter geschalteter Elektronik und eine geeignete Optik aufweist und Glasfasern, ein flexibler Lichtwellenleiter und/oder ein Polymervollstab in einem separaten Beleuchtungskanal vom Handgriff über den Schaft zur Instrumentenspitze geführt sind.

Für eine gute Ausleuchtung in-situ ist es insbesondere vorteilhaft, wenn an der Instrumentspitze LEDs zur Beleuchtung angeordnet sind.

Vorteilhaft ist ferner wenn dass der Schaft mindestens teilweise einen kreisförmigen oder elliptischen Querschnitt aufweist. Ein elliptischer Querschnitt kann für die Anordnung der Instrumente im Inneren des Schaftes vorteilhaft sein. Auch kann der Schaft u.U. etwas flacher ausgebildet werden, als dies bei einem kreisförmigen Querschnitt der Fall gewesen sein würde.

Weitere Ausführungsformen sind Gegenstand der Unteransprüche und/oder werden im Folgenden anhand von Figuren näher dargestellt, wobei
- Fig. 1: eine schematische perspektivische Darstellung einer Ausführungsform des Instrumentes zur Bearbeitung, insbesondere der Entnahme eines Gefäßes zeigt;
- Fig. 2: eine Spitze einer Ausführungsform des Instrumentes zeigt;
- Fig. 3: eine Schnittansicht durch den Handgriff einer Ausführungsform des Instruments zeigt;
- Fig. 4: die Verwendung einer Ausführungsform des Instruments zeigt;
- Fig. 5: die Spitze einer Ausführungsform mit nicht ausgefahrener Spitze zeigt;
- Fig. 5a: eine abgewandelte Ausführungsform gemäß Fig. 5 zeigt;
- Fig. 6: die Spitze einer Ausführungsform des Instrumentes mit einer axial nach distal ausgefahrener Spitze zeigt;
- Fig. 7: eine Ausführungsform mit einem Spülkanal zeigt;
- Fig. 8: eine Ausführungsform mit einem Videoanschluss zeigt;

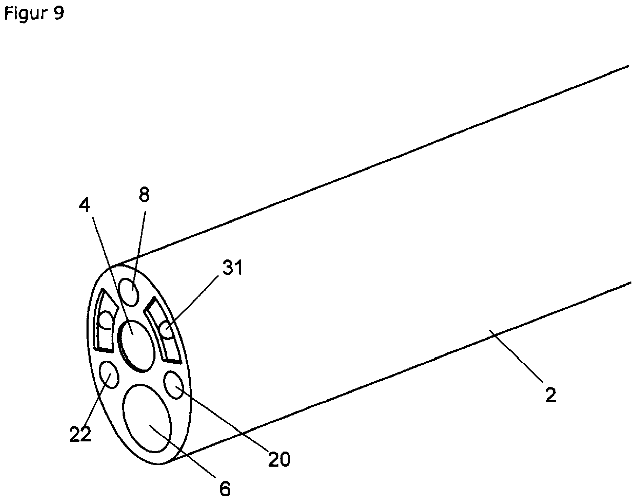

- Fig. 9: eine Ausführungsform mit einem Polymervollstab als Lichtleiter zeigt;
- Fig. 10: eine Ausführungsform mit LED an der Spitze zeigt;

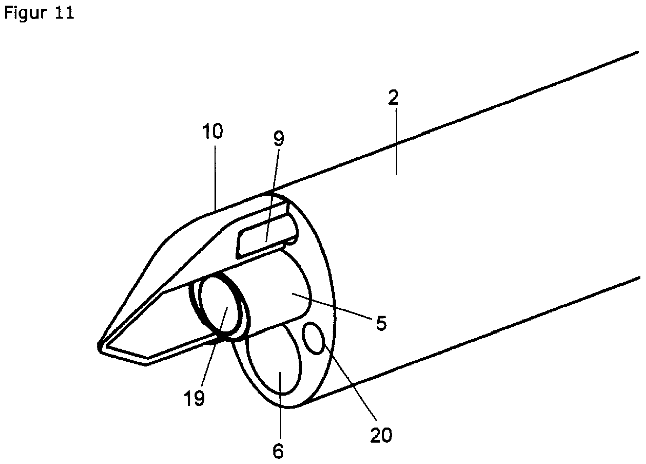

- Fig. 11: ein Detail einer weiteren Ausführungsform zeigt;
- Fig. 12: ein Detail einer weiteren Ausführungsform betreffend die Strömungsform von Spülgas zeigt;
- Fig. 13a, b: jeweils Schnittansichten durch die Spitze und den Schaft einer Ausführungsform zeigen;
- Fig. 14: eine Explosionszeichnung einer Ausführungsform einer Spitze mit einer Prallplatte zeigt.

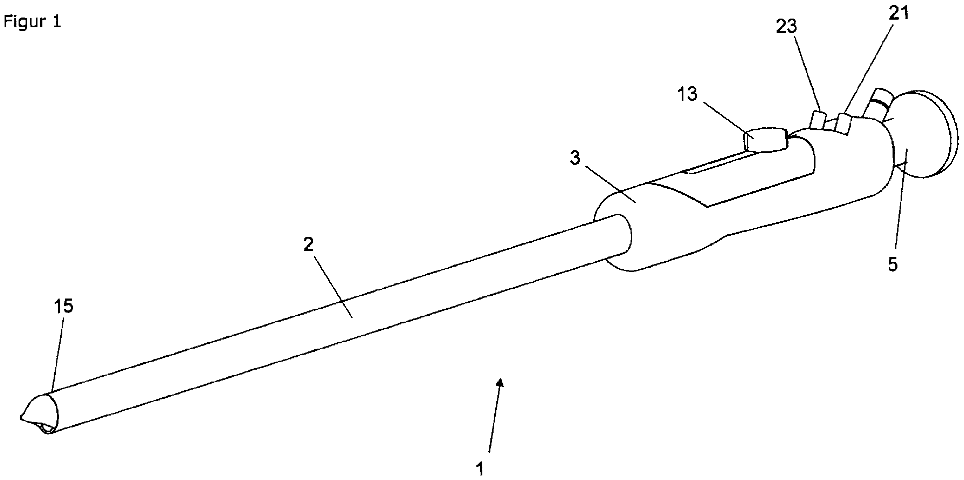

In Fig. 1 ist der Grundaufbau einer Ausführungsform eines invasiven Instruments 1, insbesondere zur Entnahme von Gefäßen 14 dargestellt.

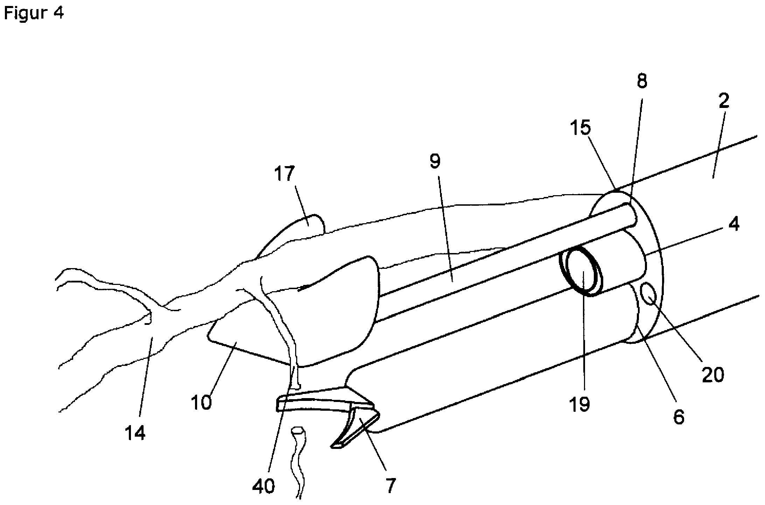

Im Folgenden wird ein Gerät beschrieben, das zur Entnahme von Gefäßen wie der Vena Saphena Magna (siehe Fig. 4) besonders ausgebildet ist. Grundsätzlich können die Abmessungen des Instruments 1 den Gegebenheiten des Körpers angepasst werden.

Andere Gefäße 14 des Menschen, die beispielsweise mit dem Instrument 1 bearbeitet werden können, sind die Vena saphena parva (Subcutane Wadenvene), die Vena cepahlica brachii & antebrachii (subcutane Vene am Ober- und Unterarm) oder die Vena basilica (tiefliegende Vene im Oberarm). Die Erfindung allerdings nicht auf die Bearbeitung dieser Gefäße 14 beschränkt.

Das Instrument 1 dient dazu, ein Gefäß 14 (auch als Hauptgefäß bezeichnet) invasiv im Körper eines Menschen (ggf. auch eines Tieres) zu bearbeiten (Dissektion), damit es aus dem Körper entnehmbar ist.

Das Instrument 1 weist einen in den Körper einzuführenden Schaft 2 und am proximalen Ende einen Handgriff 3 auf. Am distalen Ende des Schaftes 2 (Instrumentenspitze 15) ist eine Spitze 10 aus transparentem Material angeordnet, deren Funktion und Formgebung später noch erläutert wird.

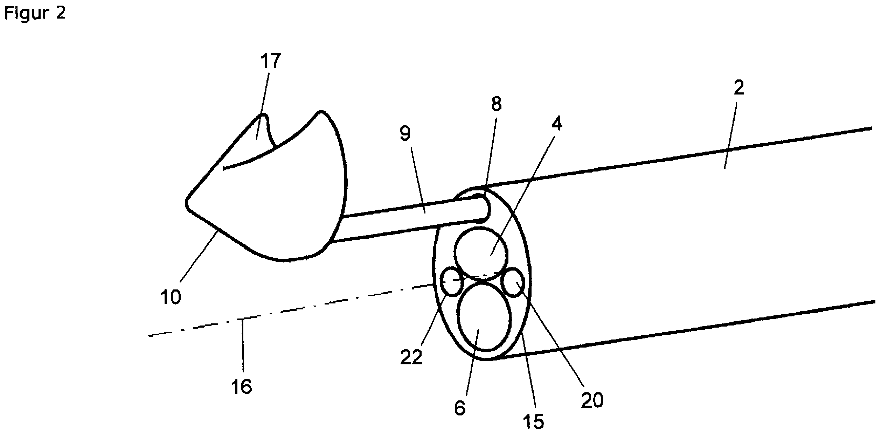

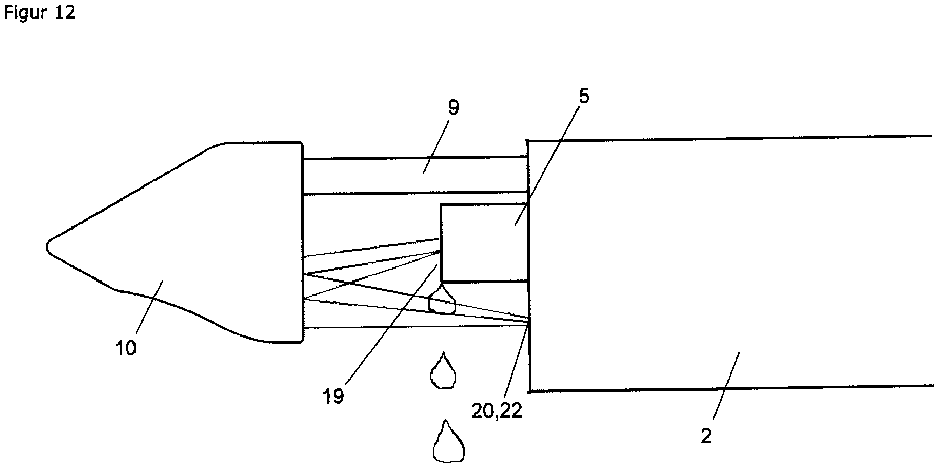

Wie im Zusammenhang mit Fig. 2 dargestellt, sind in dem Schaft 2 verschiedene Funktionseinheiten angeordnet. Im vorliegenden Beispiel sind diese Funktionseinheiten ein Endoskopkanal 4 zur Aufnahme eines Endoskops 5 (hier aus Gründen der Übersichtlichkeit nicht dargestellt), ein Arbeitskanal 6 zur Aufnahme einer Schneidvorrichtung 7 (hier aus Gründen der Übersichtlichkeit nicht dargestellt), sowie ein Führungskanal 8 zur Aufnahme eines Führungselementes 9 (z. B. in Form einer Führungsstange 9, hier aus Gründen der Übersichtlichkeit nicht dargestellt), die der Manipulation der transparenten Spitze 10 dient, angeordnet. Der Begriff Funktionseinheiten umfasst Vorrichtungen in, an und/oder am Schaft 2 des Instruments 1, mit denen insbesondere im Körper Arbeiten und/oder Beobachtungen vorgenommen werden können und/oder Teile des Instruments 1 bewegt, gereinigt (siehe z.B. Fig. 12) und/oder eingestellt werden können. Die Funktionseinheit umfasst dabei Bereiche zur Aufnahme von Vorrichtungen (z. B. Kanäle) als auch Bereiche, in denen Vorrichtungen angeordnet sind (z. B. Schneidvorrichtung 7 in Schneidkanal 6).

In alternativen Ausführungsformen können die Funktionseinheiten im Schaft 2 anders angeordnet sein oder auch in anderer Anzahl verwirklicht sein. So können z. B. zwei oder mehr Arbeitskanäle 6 vorhanden sein.

In der in Fig. 2 dargestellten Ausführungsform liegen der Arbeitskanal 6 und der Führungskanal 8 möglichst weit voneinander entfernt, d. h. an gegenüberliegenden Seiten des Schaftquerschnitts.

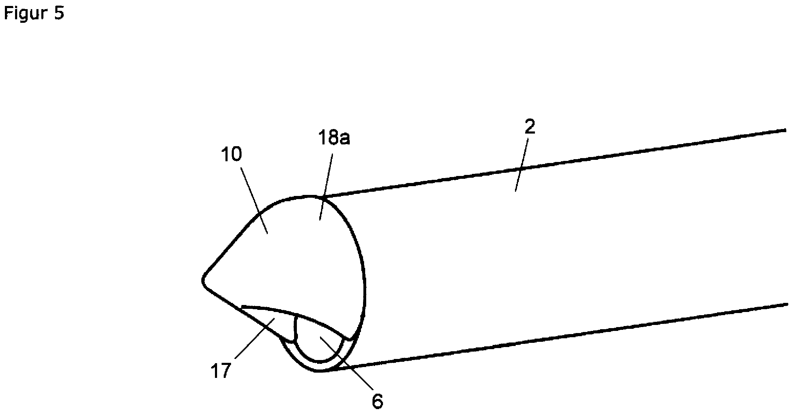

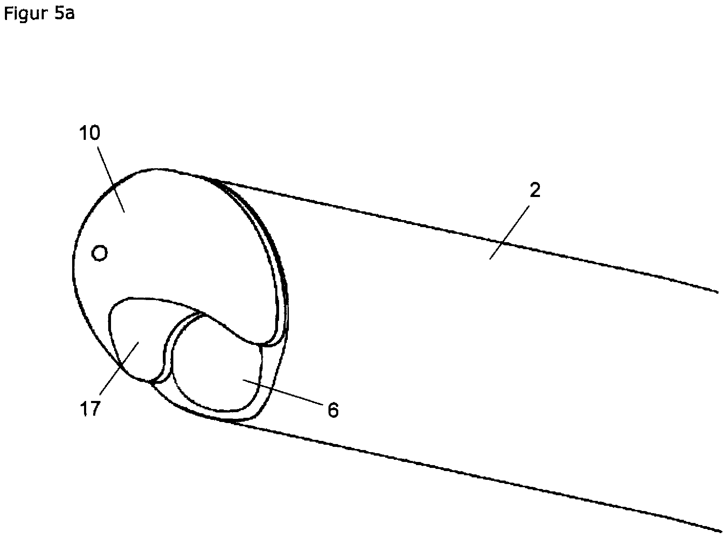

Die transparente Spitze 10 weist im Wesentlichen eine Grundstruktur mit einem kegelförmigen Anteil auf, wobei am Umfang ein Gefäßaufnahmemittel 17 in Form einer Ausnehmung (oder auch Mulde) angeordnet ist. Die Ausnehmung 17, oder in diesem Fall das Gefäßaufnahmemittel 17 mit einer anatomisch geformten Aufnahme für die Gefäße 14 hat eine Doppelfunktion, die im Zusammenhang mit den Fig. 4 und 5 noch erläutert wird.

Die Spitze 10 ist so ausgebildet, dass mit ihr beim Einführen in den Körper eine Trennung von Gewebe möglich ist. Die Spitze 10 in der hier dargestellten Ausführungsform ist leicht abgerundet, um Schäden am zu trennenden Gewebe zu vermeiden. Die Spitze 10 kann, wie später an Beispielen noch gezeigt wird, auch andere Formen annehmen.

Der Querschnitt des Schafts 2 und die Grundfläche der Spitze 10 sind in dieser Ausführungsform kreisförmig ausgebildet. Grundsätzlich ist es auch möglich, dass der Schaft 2 und die Spitze 10 polygonale oder elliptische Querschnitte aufweisen. Transparent bedeutet in diesem Fall, dass die Spitze 10 für die Wellenlängen transparent ist, unter denen eine Beobachtung stattfindet. Für eine endoskopische Beobachtung mit einer üblichen Videokamera könnten z. B. optisch transparente Polymere (z. B. PMMA) verwendet werden. Die Spitze 10 ist von Innen hohl, so dass im die Wandstärke der Spitze 10 im Wesentlichen überall gleich ist. Dies ist sinnvoll, um eine möglichst gute Beobachtbarkeit mit einem Endoskop 5 durch die transparente Spitze 10 zu gewährleisten.

Die Verbindungsstelle zwischen Führungselement 9 und transparenter Spitze 10 liegt nicht in der Mittelachse des im Wesentlichen kegelförmigen Körpers der transparenten Spitze 10, sondern seitlich versetzt. Wie in Fig. 2 erkennbar, ist die Verbindungsstelle räumlich gegenüber dem Gefäßaufnahmemittel 17 in der Nähe des Umfangs der transparenten Spitze 10 angeordnet. In der in Fig. 2 dargestellten Stellung der transparenten Spitze 10 zeigt das Gefäßaufnahmemittel 17 nach oben. Die Spitze ist so ausgebildet, dass auch in dieser Stelle mindestens eine Funktionseinheit, hier sind es gleich mehrere, freihält.

Wenn die transparente Spitze 10 mittels des Führungselementes 9 um 180° gedreht wird, zeigt das konkave Gefäßaufnahmemittel 17 nach unten. Wie in Fig. 5 zu sehen ist, ist auch in dieser Stellung der Arbeitskanal 6 frei, d. h. die Ausnehmung 17 (d. h. das Gefäßaufnahmemittel) lässt auch in dieser Stellung mindestens eine Funktionseinheit, hier den Arbeitskanal 6 frei.

Damit wird deutlich, dass das Gefäßaufnahmemittel 17 als Ausnehmung eine Doppelfunktion hat. Zum einen stellt es sicher, dass auch in der in Fig. 5 dargestellten Betriebsstellung, mindestens eine Funktionseinheit (z. B. der Arbeitskanal 6) frei bleibt. Zum anderen dient die Ausnehmung des Gefäßaufnahmittels 17 als Positionierungsmittel für das Gefäß 14 (d.h. dem Hauptgefäß), d. h. durch Rotation der Spitze 10 mit dem Gefäßaufnahmemittel 17 kann das Gefäß 14 in eine Lage gebracht werden, in der es gut bearbeitet werden kann.

In alternativen Ausführungsformen können auch mehr als ein Endoskopkanal 4, mehr als ein Arbeitskanal 6 und/oder mehr als Führungskanal 8 vorhanden sein.

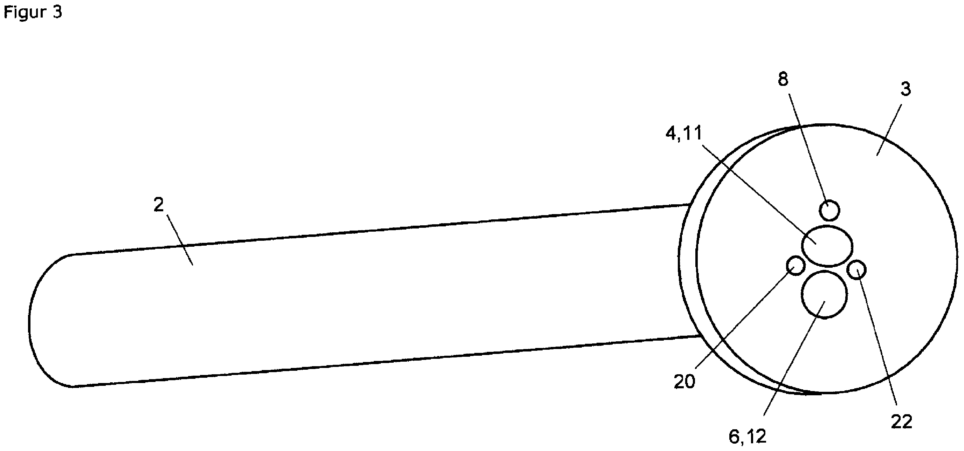

In der Schnittansicht der Fig. 3 ist erkennbar, dass am Handgriff 3 eine Öffnung 11 des Endoskopkanals 4 zur Aufnahme des Endoskops 5, eine Öffnung 12 des Arbeitskanals 6 sowie ein mit dem Führungselement 9 verbundener Manipulator 13 angeordnet sind.

Im Folgenden wird Funktion der Ausführung vor allem in Bezug auf Fig. 4 erläutert.

Wenn ein Gefäß 14 vom umliegenden Gewebe (in Fig. 4 nicht dargestellt) getrennt werden soll, so ist die durchsichtige Spitze 10 so am Schaft 2 angeordnet, dass die Grundlinie der Spitze 10 in etwa dem Umfang des Schafts entspricht (siehe z. B. Fig. 5); die Spitze 10 ist bündig mit dem Schaft 2.

Die transparente Spitze 10 ist hier derart gestaltet, dass sich ein zu entnehmendes Gefäß 14 von der Spitze 10 so aufnehmen oder halten lässt, dass es von der Schneidvorrichtung 7, die im Arbeitskanal 6 angeordnet ist, ferngehalten und / oder vor ungewollter Beschädigung geschützt wird.

Hierzu wird die transparente Spitze 10 (mit dem Gefäßaufnahmemittel 17 nach unten zeigend) mittels des Manipulators 13 (siehe Fig. 1) über das Führungselement 9 von der dem Gefäß 14 zugewandten Seite, der Instrumentenspitze 15, entlang der Instrumentenachse 16, d. h. der Längsachse des Schaftes 2 (siehe Fig. 2 und 4) wegbewegt. Dies bedeutet, dass die transparente Spitze 10 in axialer Richtung von proximal nach distal ausgefahren wird.

Der Manipulator 13 (siehe Fig. 1) fungiert hier als eine Art Schiebemechanismus, mit dem das Führungselement 9, und damit die transparente Spitze 10 im Körper in axialer Richtung bewegt werden kann und als ein Drehmechanismus (z. B. mit einem Rädchen) mit dem eine rotatorische Bewegung ausgeführt werden kann. Grundsätzlich kann der Manipulator 13 auch auf mehrere Bedienelemente verteilt werden.

Nach der Positionierung der transparenten Spitze 10 in axialer Richtung wird die transparente Spitze 10 mittels des Manipulators 13 um die Achse des Führungselementes 9 um bis zu 180° geschwenkt, so dass das Gefäß 14 in der muldenförmig gestalteten Gefäßaufnahmemittel 17 (siehe auch Fig. 2) angeordnet wird und von der Schneidvorrichtung 7 fern gehalten wird.

Die Schneidvorrichtung 7 kann dann, so wie in Fig. 4 dargestellt, z. B. Nebengefäße 40 durchtrennen, wobei sich die Schneidvorrichtung 7 in axialer Richtung frei bewegen kann. In Fig. 4 wird eine Scherenvorrichtung als Schneidvorrichtung 7 verwendet. Grundsätzlich sind aber auch andere Schneidmittel denkbar.

In alternativen Ausführungsformen kann das Gefäßaufnahmemittel 17 als Kerbe oder als U-förmiger Einschnitt ausgebildet sein. In jedem Fall ist es möglich, das Gefäß 14 in einem Gefäßaufnahmemittel 17 so zu lagern, dass es seitlich nicht so leicht herausrutscht.

Es ist möglich, die axiale Distanz zwischen der ausgefahrenen transparenten Spitze 10 und der Instrumentenspitze 15 mittels des Manipulators 13 und der Führungsstange 9 stufenlos oder in diskreten Schritten einzustellen.

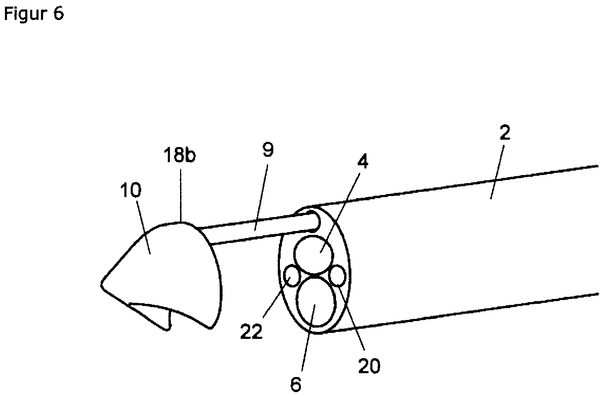

Mit der dargestellten Ausführungsform ist es möglich, dass die transparente Spitze 10 derart gestalten ist, dass sich die Schneidvorrichtung 7 sowohl in eingefahrener Position 18a (siehe Fig. 5) als auch ausgefahrener Position 18b (siehe Fig. 6) an der Spitze 10 vorbei führen lässt. In den Fig. 5 und 6 ist dargestellt, dass das Gefäßaufnahmemittel 17 in der Spitze 10 so ausgebildet ist, dass die muldenartige Ausbildung den Arbeitskanal 6 nicht verdeckt.

Dies erlaubt dem Operateur die Präparation von Gefäßen 14, Nebengefäßen 40 und auch von umliegendem Gewebe während jeder Phase des Eingriffs. Die Spitze 10 ist so ausgebildet, dass in jeder Phase eine Funktionseinheit, hier der Arbeitskanal 6 mit der darin angeordneten Schneidvorrichtung 7, arbeitsfähig ist.

Der Operateur kann die Schneidvorrichtung 7 sogar axial über die Spitze 10 weiter distal hinaus bewegen, um vor dem eigentlichen Instrument 1 und / oder der Spitze 10 zu schneiden. Das Gefäßaufnahmemittel 17 kann dann dazu verwendet werden, um das Gefäß 14 räumlich in einer Lage zu fixieren, in der ein Operateur besonders gut arbeiten kann. Dabei erlaubt die Drehbarkeit des Führungselementes 9 eine beliebige Positionierung, wobei in jedem Fall der notwendige Abstand zu der Schneidvorrichtung 7 eingehalten wird. Durch die axiale Verschiebbarkeit und / oder Drehbarkeit (d.h. Bewegungen relativ zum Schaft 2) ist es auch möglich, das Gefäß 14 mit den anhängenden Nebengefäßen 40 so auszurichten, dass ein Präparieren oder Abtrennen des Nebengefäßes 40 möglich ist.

Damit ist insbesondere die Gefäßentnahme ohne Wechsel des Instrumentes und / oder Entfernen der transparenten Spitze 10 möglich.

Fig. 5a zeigt eine Abwandlung einer Ausführungsform, die in Fig. 5 dargestellt ist, in einer etwas anderen Perspektive. Das Gefäßaufnahmemittel 17 ist hier nach unten gedreht, so dass die Funktionseinheit, in diesem Fall der der Arbeitskanal 6, in axialer Richtung durch die Ausnehmung des Gefäßaufnahmemittels 17 frei liegt. Somit kann die Spitze 10 mit der Ausnehmung 17 nicht nur zum Halten oder Lagern von Gefäßen 14 verwendet werden, sondern die Spitze 10 ist durch die Ausnehmung 17 auch so gestaltet, dass die Funktionseinheiten freiliegen.

In einem weiteren Ausführungsbeispiel des Instruments 1 werden die Komponenten Handgriff 3 und / oder Schaft 2 als Mehrweginstrument ausgeführt. Diese werden derart gestaltet, dass sie sich maschinell aufbereiten lassen und anschließend mit gängigen Sterilisationsverfahren, insbesondere der Dampfsterilisation, sterilisieren lassen. Die Spitze 10, das Führungselement 9 und / oder der Manipulator 13 können dabei als Einwegkomponenten ausgeführt sein und können durch modulare Bauweise einfach in das Mehrweginstrument angebracht und wieder entfernt werden.

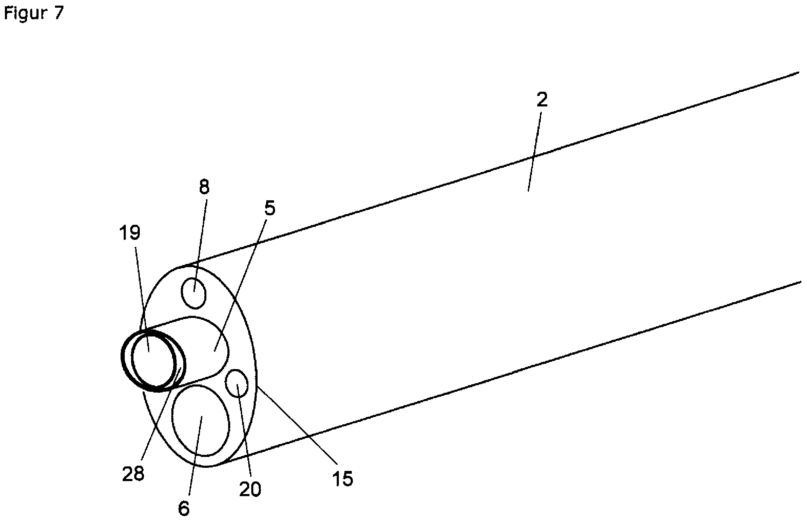

In einer weiteren Ausführungsform (siehe Fig. 7) ist in dem Schaft 2 ein Spülkanal 20 für die Zufuhr und Absaugung von Spül- und Körperflüssigkeiten angeordnet, so dass sich eine Spitze 19 des Endoskops 5 und die transparente Spitze 10 mittels einer Spülung in-situ reinigen lassen. Der Effekt der Reinigung wird verstärkt, wenn - wie in Fig. 12 dargestellt, die Endoskopspitze 19 aus der Instrumentenspitze 15 heraus und in die eingefahrene transparente Spitze 10 hineinragt. Der Spülkanal 20 endet im Handgriff in einem Anschluss 21 für die Zufuhr und Absaugung von Spül- und Körperflüssigkeiten (siehe Fig. 1).

Ein weiteres Ausführungsbeispiel (siehe Fig. 12) erlaubt, dass CO₂ Gas (oder auch ein anderes Gas) für die Insufflation in einem integrierten Insufflationskanal 22 als Funktionseinheit bis zur Instrumentenspitze 15 geleitet wird. Die Position des Insufflationskanals 22 ist zur Endoskopspitze 19 derart anzuordnen, dass sich mit dem CO₂ Gas die Endoskopspitze von unerwünschter Flüssigkeit befreien lässt. Dies kann beispielhaft dadurch realisiert werden, dass der CO₂ Gasstrom an der Rückseite der transparenten Spitze 10 umgelenkt und auf die Endoskopspitze 19 gerichtet wird. Der Kanal 22 endet im Handgriff in einem Anschluss 23 (siehe Fig. 1). In Fig. 12 ist auch eine Spülfunktion dargestellt, mit der aus einem Spülkanal 20 eine Spülflüssigkeit gesprüht wird. Spülkanal 20 und Insufflationskanal 22 sind in der Seitenansicht der der Fig. 12 nur seitlich am Austritt der Strahlen erkennbar. In einem weiteren Ausführungsbeispiel ist das Instrument 1 als Einweginstrument ausgeführt, wobei das Instrument 1 grundsätzlich die gleiche Form und Funktion haben kann, wie zuvor beschrieben. Hierzu werden die Komponenten Handgriff 3 und Schaft 2 als Einwegkomponenten gestaltet. Konsequenterweise sind bei diesem Ausführungsbeispiel die Spitze 10, das Führungselement 9 und / oder der Manipulator 13 feste, nicht lösbare Bestandteile des Einweginstruments. Ebenfalls kann in das Einweginstrument ein Insufflationskanal 22 für die Insufflation und ein Arbeitskanal für die Spülung und / oder Absaugung 20 integriert werden.

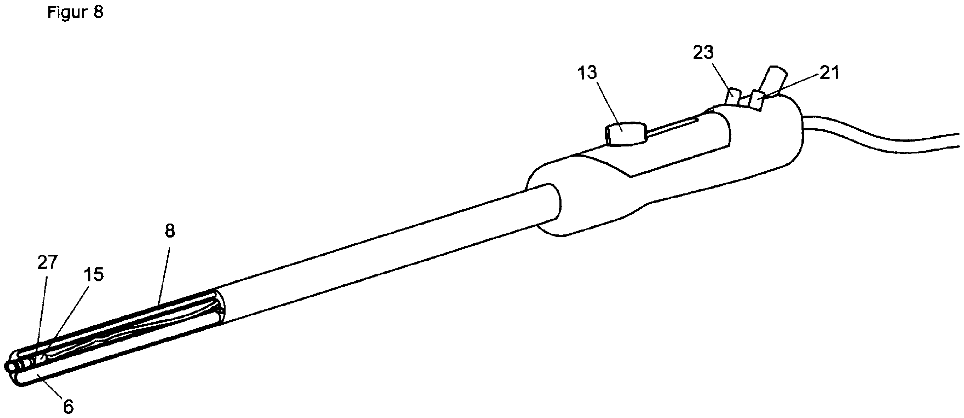

In einem weiteren Ausführungsbeispiel (Fig. 8) kann das Endoskop 5 durch eine an der Instrumentenspitze 15 integrierte Videokamera 27 ersetzt werden oder kann mit ihr zusammen verwendet werden. Diese Videokamera kann einen Videosensor, z.B. CCD oder CMOS Sensor, nebst dahinter geschalteter Elektronik und einer geeigneten Optik aufweisen. Die bei dem Endoskop 5 üblicherweise integrierten Glasfasern 28, die der Übertragung von Licht zur Beleuchtung des Operationsfeldes dienen, werden in dem hier beschriebenen Ausführungsbeispiel in einem separaten Beleuchtungskanal 29 vom Handgriff 3 über den Schaft 2 zur Instrumentenspitze 15 geführt.

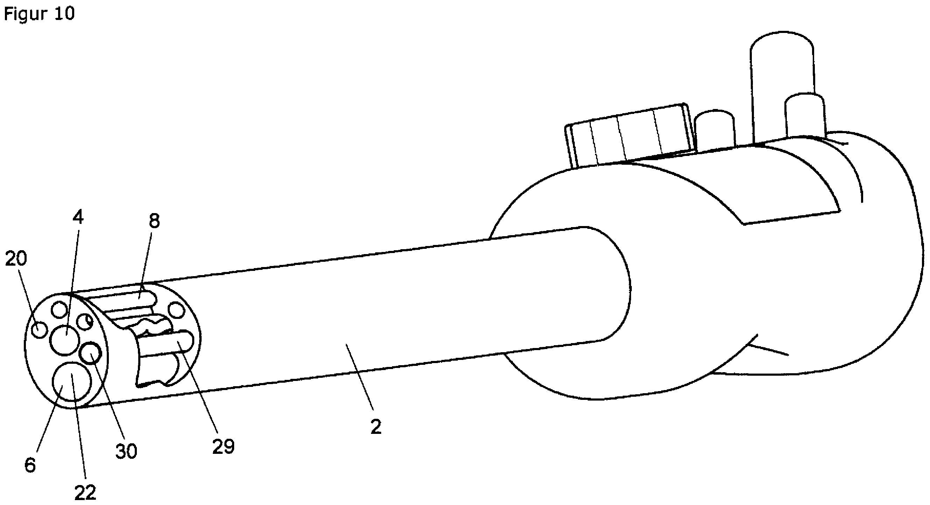

In einer weiteren Ausführungsform (Fig. 10) werden in dem Einweginstrument die Glasfasern 28 durch einen Polymervollstab 30 ersetzt. Dieser kann in einer beliebigen, platzsparenden Form, die sich zur Leitung von Licht eignet ausgeführt werden. Alternativ oder auch zusätzlich kann ein flexibler Lichtwellenleiter aus Silikon oder einem anderen geeigneten Material verwendet werden.

In einem weiteren Ausführungsbeispiel werden in dem Einweginstrument die Glasfasern 28 durch an der Instrumentspitze 15 positionierte LEDs 31 ersetzt. Grundsätzlich sind auch Kombinationsmöglichkeiten denkbar.

Diese können in einer beliebigen, platzsparenden Form, die sich zur Ausleuchtung des Sichtfeldes der Videokamera 27 eignet, ausgeführt werden.

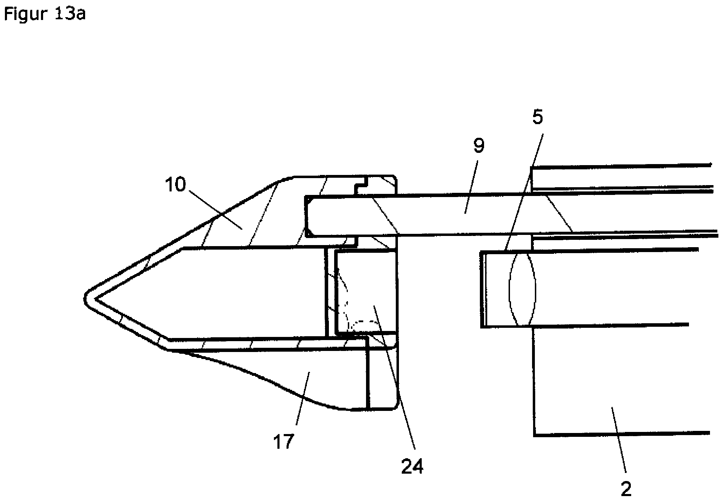

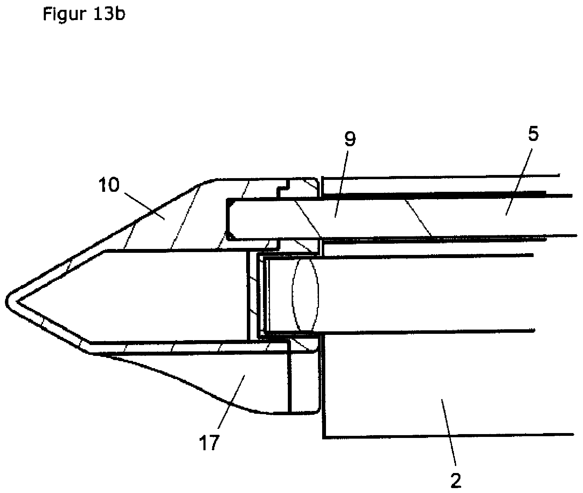

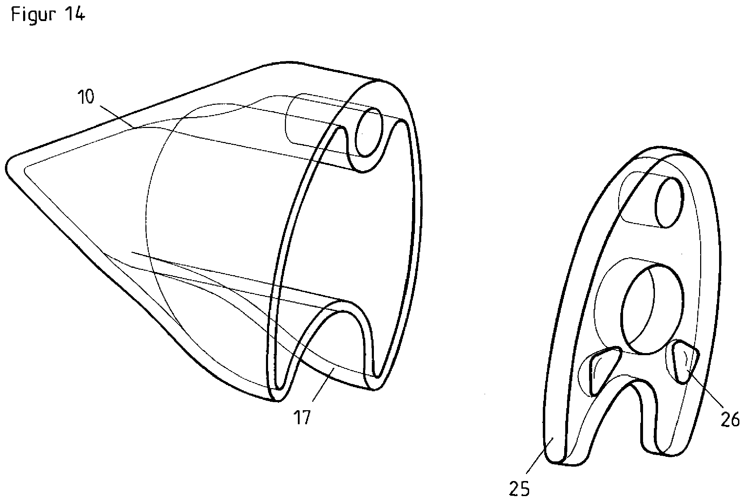

In Fig. 13a, b sind Schnittansichten durch eine transparente Spitze 10 und einen Schaft 2 dargestellt, wobei als Funktionseinheit hier ein Endoskop 5 dargestellt ist. In der Fig. 13a ist die transparente Spitze 10 in der distalen Position dargestellt. Dabei sind ein einer Aussparung 24 einige Wassertropfen dargestellt, die z.B. nach dem Spülen an der Innenseite der Spitze 10 verblieben sind. Die Aussparung 24 kann Teil einer Prallplatte sein, die am proximalen Ende der Spitze 10 angeordnet ist. Eine weitere Ausführungsform dieser proximalen Prallplatte ist in Fig. 14 dargestellt.

Wenn nun die Spitze 10 in die proximale Lage gebracht wird (Fig. 13b) ragt die Spitze des Endoskops 5 in die Aussparung 24. Das vorstehende Ende des Endoskops 5 drückt die Wassertropfen in der Aussparung 14 zusammen, so dass ein im Wesentlichen gleichmäßiger Film in dem Spalt zwischen Aussparung 24 und dem Endoskop 5 entsteht. Damit werden die optischen Eigenschaften nicht beeinträchtigt. Des Weiteren ist in den Fig. 13a,b erkennbar, dass das Innere der Spitze im Wesentlichen hohl ausgebildet ist, vor allem in dem Bereich, der axial im Blickfeld des Endoskops 5 liegt.

In Fig. 14 ist eine Spitze 10 dargestellt in Form einer Explosionszeichnung dargestellt, die als Freiform gebildet ist. Der Durchmesser verjüngt sich von proximal nach distal. Die Ausnehmung 17 weist nach unten. Das Innere der Spitze 10 ist hier im Wesentlichen hohl ausgebildet, wobei die Wandstärken des transparenten Materials im Wesentlichen gleich sind.

Am proximalen Ende der transparenten Spitze 10 ist eine Prallplatte 25 angeordnet, an der z.B. Spülflüssigkeit gemäß dem Vorgehen in Fig. 12 umgelenkt werden kann, um gezielt ein Endoskop 5 zu reinigen. Um diese Reinigung zu unterstützen, weist die Prallplatte 25 mindestens ein Umlenkmittel 26 auf, mit dem der Spülstrahl gezielt auf das Endoskop 5 gerichtet werden kann. Das Umlenkmittel 26 ist hier als Mulde ausgebildet. Grundsätzlich können auch andere Formen, die z.B. über die Grundfläche der Prallplatte 25 hinausragen zum Einsatz kommen. Auch können zusätzlich oder alternative andere Funktionseinheiten, wie z.B. die Schneidvorrichtung 6 durch den Spülstrahl gereinigt werden.

### Bezugszeichenliste

- 1: Instrument
- 2: Schaft
- 3: Handgriff
- 4: Endoskopkanal
- 5: Endoskop
- 6: Arbeitskanal
- 7: Schneidvorrichtung
- 8: Führungskanal
- 9: Führungsvorrichtung
- 10: Transparente Spitze
- 11: Öffnung des Endoskopkanals
- 12: Öffnung des Arbeitskanals
- 13: Manipulator der Führungsvorrichtung
- 14: Gefäß
- 15: Instrumentenspitze
- 16: Instrumentenachse
- 17: Gefäßaufnahmemittel
- 18a: eingefahrene Position der Spitze
- 18b: ausgefahrene Position der Spitze
- 19: Endoskopspitze
- 20: Spülkanal
- 21: Anschluss Spülkanal
- 22: Insufflationskanal
- 23: Anschluss Gaskanal
- 24: Aussparung
- 25: Prallplatte
- 26: Umlenkmittel
- 27: Videokamera
- 28: Glasfasern
- 29: Beleuchtungskanal
- 30: Polymervollstab
- 31: LED
- 40: Nebengefäß

## Patentansprüche

1. Invasives Instrument zur Bearbeitung eines Gefäßes, das ggf. Nebengefäße aufweist, mit einer Spitze (10) am distalen Ende eines in den Körper einzuführenden Schaftes (2) und mindestens einer Funktionseinheit (4, 5, 6, 7, 8, 9, 20, 22),
**dadurch gekennzeichnet, dass**
die Spitze (10) zur Dissektion von Gewebe ausgebildet ist und mindestens ein Gefäßaufnahmemittel (17) aufweist, das so angeordnet und ausgebildet ist, dass im Betriebszustand des Instruments (1) stets die mindestens eine Funktionseinheit (4, 5, 6, 7, 8, 9, 20, 22) des Schaftes (2) freigelassen ist, so dass Arbeiten am Gefäß (14),
einem Nebengefäß (40) und / oder am umliegenden Gewebe mit oder durch die Funktionseinheit (4, 5, 6, 7, 8, 9, 20, 22) möglich sind und mit dem mindestens einen Gefäßaufnahmemittel (17) das Gefäß (14) in einer räumlichen Position fixierbar ist, wobei die Spitze (10) mittels eines Führungselementes (9) relativ zum Schaft (2) bewegbar ist und die Spitze (10) in axialer Richtung verschiebbar ist, so dass im Eingriff ein im mindestens einen Gefäßaufnahmemittel (17) angeordnetes Gefäß (14) von der Funktionseinheit (4, 5, 6, 7, 8, 9, 20, 22) gezielt beabstandbar ist
und dass eine Endoskopspitze (19) aus dem distalen Endes des Schafts (2) heraus und in die eingefahrene Spitze (10) hineinragt und die Spitze (10) transparent ist.

2. Invasives Instrument nach Anspruch 1, **dadurch gekennzeichnet, dass** die Spitze (10) mindestens teilweise einen kegelförmigen Bereich, einen prismatischen Bereich, einen Bereich in der Form einer Delfinnase, einen Bereich mit der Form eines Kegelstumpfs und / oder einen Bereich mit der Form eines Dreikants aufweist.

3. Invasives Instrument nach Anspruch 1 oder 2, **dadurch gekennzeichnet, dass** die Spitze (10) um bis zu 180° um die Längsachse verschwenkbar ist, so dass im Eingriff ein im mindestens einen Gefäßaufnahmemittel (17) angeordnetes Gefäß (14) von einer Schneidvorrichtung (7) gezielt beabstandbar ist.

4. Invasives Instrument nach mindestens einem der vorhergehenden Ansprüche, **gekennzeichnet durch** einen Manipulator (13), insbesondere an einem Handgriff (3) angeordnet, zur axialen Verschiebung und / oder Rotation der Spitze (10).

5. Invasives Instrument nach mindestens einem der vorhergehenden Ansprüche, **dadurch gekennzeichnet, dass** das Führungselement (9) im Wesentlichen stabförmig ausgebildet ist und an einer Stelle der Spitze (10) befestigt ist, die dem Gefäßaufnahmemittel (17) gegenüberliegt.

6. Invasives Instrument nach mindestens einem der vorhergehenden Ansprüche, **dadurch gekennzeichnet, dass** der Führungskanal (8) und der Arbeitskanal (6) auf gegenüberliegenden Seiten des Schaftes (2) angeordnet sind.

7. Invasives Instrument nach mindestens einem der vorhergehenden Ansprüche, **dadurch gekennzeichnet, dass** im Schaft (2) und / oder im Handgriff (3) des Instruments (1) mindestens ein Endoskopkanal (4), mindestens ein Arbeitskanal (6) zur Aufnahme einer Schneidvorrichtung (7), mindestens ein Führungskanal (8) zur Aufnahme eines Führungselementes (9), mindestens ein Spülkanal (20) und / oder mindestens ein Insufflationskanal (22) angeordnet sind, insbesondere dass der Handgriff (3) und / oder der Schaft (2) als Mehrweginstrument ausgeführt sind.

8. Invasives Instrument nach mindestens einem der vorhergehenden Ansprüche, **dadurch gekennzeichnet, dass** Spitze (10) mit der Ausnehmung derart gestaltet ist, dass sich die Schneidvorrichtung (7) und / oder ein Endoskop (5) in axial eingefahrener Position(18a) und / oder auch axial ausgefahrener (18b) Position an der Spitze (10) vorbeischieben lässt.

9. Invasives Instrument nach mindestens einem der vorhergehenden Ansprüche, **dadurch gekennzeichnet, dass** die Distanz zwischen ausgefahrener Spitze (10) und Instrumentenspitze (15) stufenlos oder in diskreten Schritten einstellbar ist.

10. Invasives Instrument nach mindestens einem der vorhergehenden Ansprüche, **dadurch gekennzeichnet, dass** die Spitze (10), die Führungsstange (9) und / oder der Manipulator (13) als Einwegkomponenten ausgeführt sind.

11. Invasives Instrument nach mindestens einem der vorhergehenden Ansprüche, **dadurch gekennzeichnet, dass** in den Schaft (2) mindestens ein Kanal (20) zur Leitung von Spülflüssigkeit und / oder zur Absaugung unerwünschter Flüssigkeiten integriert ist, insbesondere dass der Schaft (2) mindestens teilweise einen kreisförmigen oder elliptischen Querschnitt aufweist.

12. Invasives Instrument nach mindestens einem der vorhergehenden Ansprüche, **dadurch gekennzeichnet, dass** Gas, insbesondere CO₂ Gas für die Insufflation in einem Insufflationskanal (22) bis zur Instrumentenspitze (15) leiten lässt.

13. Invasives Instrument nach mindestens einem der vorhergehenden Ansprüche, **dadurch gekennzeichnet, dass** der Insufflationskanal (22) relativ zur Endoskopspitze (19) derart angeordnet ist, dass sich mit dem Gas die Endoskopspitze (19) reinigen lässt, insbesondere indem der Gasstrom an der Rückseite der Spitze (10) umgelenkt und auf die Endoskopspitze (19) gerichtet wird.

14. Invasives Instrument nach mindestens einem der vorhergehenden Ansprüche, **dadurch gekennzeichnet, dass** die Spitze (10) mindestens ein Umlenkmittel (26) für einen Spülstrahl aufweist.

15. Invasives Instrument nach mindestens einem der vorhergehenden Ansprüche, **dadurch gekennzeichnet, dass** an der Instrumentenspitze (15) eine Videokamera (27) angeordnet ist, wobei die Videokamera (27) einen Videosensor, z.B. einen CCD oder CMOS Sensor, nebst dahinter geschalteter Elektronik und eine geeignete Optik aufweist und Glasfasern (28), ein flexibler Lichtwellenleiter und / oder ein Polymervollstab (30) in einem separaten Beleuchtungskanal (29) vom Handgriff (3) über den Schaft (2) zur Instrumentenspitze (15) geführt sind und / oder dass an der Instrumentspitze (15) LEDs (31) zur Beleuchtung angeordnet sind.

## Claims

1. An invasive instrument for treating a vessel, which possibly includes secondary vessels, with a tip (10) at the distal end of a shaft (2) to be introduced into the body and at least one functional unit (4, 5, 6, 7, 8, 9, 20, 22),
**characterized in that**
the tip (10) is formed for the dissection of tissue and includes at least one vessel receiving means (17) which is arranged and formed such that in the operating condition of the instrument (1) always the at least one functional unit (4, 5, 6, 7, 8, 9, 20, 22) of the shaft (2) is kept clear, so that working on the vessel (14), a secondary vessel (40) and/or on the surrounding tissue is possible using or by means of the functional unit (4, 5, 6, 7, 8, 9, 20, 22) and with the at least one vessel receiving means (17) the vessel (14) can be fixed in a spatial position, wherein the tip (10) is movable relative to the shaft (2) by means of a guide element (9) and the tip (10) is shiftable in axial direction so that during the procedure a vessel (14) arranged in the at least one vessel receiving means (17) can be spaced selectively by the functional unit (4, 5, 6, 7, 8, 9, 20, 22) and an endoscope tip (19) protrudes out of the distal end of a shaft (2) and into the retracted tip (10) and the tip (10) is transparent.

2. The invasive instrument according to claim 1, **characterized in that** the tip (10) at least partly includes a conical region, a prismatic region, a region in the form of a dolphin nose, a region with a frustoconical shape and/or a region with the shape of a triangle.

3. The invasive instrument according to claim 1 or 2, **characterized in that** the tip (10) is pivotable about the longitudinal axis by up to 180°, so that during the procedure a vessel (14) arranged in the at least one vessel receiving means (17) can be spaced selectively from a cutting device (7).

4. The invasive instrument according to at least one of the preceding claims, **characterized by** a manipulator (13), in particular arranged at a handle (3), for axially shifting and/or rotating the tip (10).

5. The invasive instrument according to at least one of the preceding claims, **characterized in that** the guide element (9) is formed substantially rod-shaped and is attached at a point of the tip (10) which is located opposite the vessel receiving means (17).

6. The invasive instrument according to at least one of the preceding claims, **characterized in that** the guide channel (8) and the working channel (6) are arranged on opposite sides of the shaft (2).

7. The invasive instrument according to at least one of the preceding claims, **characterized in that** in the shaft (2) and/or in the handle (3) of the instrument (1) at least one endoscope channel (4), at least one working channel (6) for receiving a cutting device (7), at least one guide channel (8) for receiving a guide element (9), at least one irrigation channel (20) and/or at least one insufflation channel (22) are arranged, in particular the handle (3) and/or the shaft (2) are designed as reusable instrument.

8. The invasive instrument according to at least one of the preceding claims, **characterized in that** the tip (10) with the recess is designed such that the cutting device (7) and/or an endoscope (5) can be moved past the tip (10) in an axially retracted position (18a) and/or also in an axially extended position (18b).

9. The invasive instrument according to at least one of the preceding claims, **characterized in that** the distance between extended tip (10) and instrument tip (15) is adjustable steplessly or in discrete steps.

10. The invasive instrument according to at least one of the preceding claims, **characterized in that** the tip (10), the guide rod (9) and/or the manipulator (13) are designed as disposable components.

11. The invasive instrument according to at least one of the preceding claims, **characterized in that** into the shaft (2) at least one channel (20) is integrated for conducting rinsing liquid and/or for sucking off undesired liquids, in particular the shaft (2) has an at least partly circular or elliptical cross-section.

12. The invasive instrument according to at least one of the preceding claims, **characterized in that** gas, in particular CO₂ gas for the insufflation can be passed to the instrument tip (15) in an insufflation channel (22).

13. The invasive instrument according to at least one of the preceding claims, **characterized in that** the insufflation channel (22) is arranged relative to the endoscope tip (19) such that the endoscope tip (19) can be cleaned with the gas, in particular **in that** the gas stream is diverted on the back of the tip (10) and directed to the endoscope tip (19).

14. The invasive instrument according to at least one of the preceding claims, **characterized in that** the tip (10) includes at least one diverting means (26) for an irrigation jet.

15. The invasive instrument according to at least one of the preceding claims, **characterized in that** at the instrument tip (15) a video camera (27) is arranged, wherein the video camera (27) includes a video sensor, e.g. a CCD or CMOS sensor, along with associated electronics and suitable optics, and glass fibers (28), a flexible optical waveguide and/or a solid polymer rod (30) are guided in a separate illumination channel (29) from the handle (3) via the shaft (2) to the instrument tip (15) and/or that at the instrument tip (15) LEDs (31) are arranged for illumination.

## Revendications

1. Instrument invasif pour le traitement d'un vaisseau, qui présente le cas échéant des vaisseaux secondaires, avec une pointe (10) à l'extrémité distale d'une tige (2) à introduire dans le corps et au moins une unité fonctionnelle (4, 5, 6, 7, 8, 9, 20, 22), **caractérisé en ce que**
la pointe (10) est réalisée pour la dissection de tissu et présente au moins un moyen de réception de vaisseau (17), qui est agencé et réalisé de sorte que l'au moins une unité fonctionnelle (4, 5, 6, 7, 8, 9, 20, 22) de la tige (2) est toujours libérée à l'état de fonctionnement de l'instrument (1), si bien que des interventions sur le vaisseau (14), un vaisseau secondaire (40) et/ou sur le tissu environnant sont possibles avec ou par l'unité fonctionnelle (4, 5, 6, 7, 8, 9, 20, 22) et le vaisseau (14) peut être fixé dans une position spatiale avec l'au moins un moyen de réception de vaisseau (17), dans lequel la pointe (10) est mobile par rapport à la tige (2) au moyen d'un élément de guidage (9) et la pointe (10) peut être déplacée dans la direction axiale, si bien qu'en prise un vaisseau (14) agencé dans l'au moins un moyen de réception de vaisseau (17) peut être espacé de manière ciblée de l'unité fonctionnelle (4, 5, 6, 7, 8, 9, 20, 22)
et **en ce qu'**une pointe endoscopique (19) fait saillie de l'extrémité distale de la tige (2) et pénètre dans la pointe rentrée (10) et la pointe (10) est transparente.

2. Instrument invasif selon la revendication 1, **caractérisé en ce que** la pointe (10) présente au moins en partie une zone conique, une zone prismatique, une zone en forme de nez de dauphin, une zone en forme de cône tronqué et/ou une zone en forme de triangle.

3. Instrument invasif selon la revendication 1 ou 2, **caractérisé en ce que** la pointe (10) peut être pivotée jusqu'à 180° autour de l'axe longitudinal, si bien qu'en prise un vaisseau (14) agencé dans l'au moins un moyen de réception de vaisseau (17) peut être espacé de manière ciblée d'un dispositif de coupe (7).

4. Instrument invasif selon au moins l'une quelconque des revendications précédentes, **caractérisé par** un manipulateur (13), agencé en particulier au niveau d'une poignée (3), pour le déplacement axial et/ou la rotation de la pointe (10).

5. Instrument invasif selon au moins l'une quelconque des revendications précédentes, **caractérisé en ce que** l'élément de guidage (9) est réalisé sensiblement en forme de baguette et est fixé à un endroit de la pointe (10), qui fait face au moyen de réception de vaisseau (17).

6. Instrument invasif selon au moins l'une quelconque des revendications précédentes, **caractérisé en ce que** le canal de guidage (8) et le canal de travail (6) sont agencés sur des côtés opposés de la tige (2).

7. Instrument invasif selon au moins l'une quelconque des revendications précédentes, **caractérisé en ce qu'**au moins un canal endoscopique (4), au moins un canal de travail (6) pour la réception d'un dispositif de coupe (7), au moins un canal de guidage (8) pour la réception d'un élément de guidage (9), au moins un canal de rinçage (20) et/ou au moins un canal d'insufflation (22) sont agencés dans la tige (2) et/ou dans la poignée (3) de l'instrument (1), en particulier **en ce que** la poignée (3) et/ou la tige (2) sont réalisées sous forme d'instrument réutilisable.

8. Instrument invasif selon au moins l'une quelconque des revendications précédentes, **caractérisé en ce que** la pointe (10) est réalisée avec l'évidement de telle manière que le dispositif de coupe (7) et/ou un endoscope (5) peut passer devant la pointe (10) en position axialement rentrée (18a) et/ou aussi en position axialement déployée (18b).

9. Instrument invasif selon au moins l'une quelconque des revendications précédentes, **caractérisé en ce que** la distance entre la pointe déployée (10) et la pointe d'instrument (15) est réglable en continu ou en pas discrets.

10. Instrument invasif selon au moins l'une quelconque des revendications précédentes, **caractérisé en ce que** la pointe (10), la barre de guidage (9) et/ou le manipulateur (13) sont réalisés sous forme de composants jetables.

11. Instrument invasif selon au moins l'une quelconque des revendications précédentes, **caractérisé en ce qu'**au moins un canal (20) est intégré dans la tige (2) pour la conduction de fluide de rinçage et/ou pour l'aspiration de fluides indésirables, en particulier **en ce que** la tige (2) présente au moins en partie une section transversale circulaire ou elliptique.

12. Instrument invasif selon au moins l'une quelconque des revendications précédentes, **caractérisé en ce que** du gaz, en particulier du CO₂ peut être conduit pour l'insufflation dans un canal d'insufflation (22) jusqu'à la pointe d'instrument (15).

13. Instrument invasif selon au moins l'une quelconque des revendications précédentes, **caractérisé en ce que** le canal d'insufflation (22) est agencé par rapport à la pointe endoscopique (19) de telle manière que la pointe endoscopique (19) peut être nettoyée avec le gaz, en particulier par le fait que le flux de gaz est dévié au niveau de la face arrière de la pointe (10) et dirigé sur la pointe endoscopique (19).

14. Instrument invasif selon au moins l'une quelconque des revendications précédentes, **caractérisé en ce que** la pointe (10) présente au moins un moyen de renvoi (26) pour un jet de rinçage.

15. Instrument invasif selon au moins l'une quelconque des revendications précédentes, **caractérisé en ce qu'**une caméra vidéo (27) est agencée au niveau de la pointe d'instrument (15), dans lequel la caméra vidéo (27) présente un capteur vidéo, par exemple un capteur CCD ou CMOS, en plus d'une électronique branchée derrière et un système optique adapté et des fibres de verre (28), une fibre optique souple et/ou une baguette pleine en polymère (30) sont guidées dans un canal d'éclairage (29) séparé de la poignée (3) à la pointe d'instrument (15) en passant par la tige (2) et/ou **en ce que** des LED (31) sont agencées au niveau de la pointe d'instrument (15) pour l'éclairage.
